# EUROPEAN PATENT APPLICATION

(11) **EP 2 311 995 A1**
(43) Date of publication of application: **20.04.2011**
(21) Application number: 10185146.7
(22) Date of filing: 22.09.2006
(51) Int. Cl.: C12Q 1/68

(54) **Methods of using IL-21**

(30) Priority: 22.09.2005 US 719388 P
(62) Divisional of application: 06792239.3
(71) Applicant: INSERM (Institut National de la Santé et de la Recherche Médicale), 75654 Paris Cedex 13 (FR)
(72) Inventor: Yssel, Hans, F-34160, Sussargues (FR); Gugliemi, Laurence, F-34070, Montpellier (FR); Pene, Jerome, F-34000, Montpellier (FR)
(74) Representative: Griffin, Philippa Jane

(57) **Abstract**

There is provided an *in vitro* method of predicting a differential therapeutic response to IL-21 in an individual in need of IL-21 therapy, comprising detecting, in a biological sample from the patient, an allelic variant of the individual's IL-21 receptor gene.

## Description

### BACKGROUND OF THE INVENTION

IL-21 is a T cell derived cytokine and has been shown to be a potent modulator of cytotoxic T cells and NK cells. (Parrish-Novak, et al. Nature 408:57-63, 2000; Parrish-Novak, et al., J. Leuk Bio. 72:856-863, 2002; Collins et al., Inmunol. Res. 28:131-140, 2003; Brady, et al. J. immunol. 172(4):2048-58, 2004.) IL-21 has been shown to co-stimulate the expansion of NK cells, and it has been demonstrated to enhance the effector functions of these cells. T cell responses include enhancement of primary antigen response as modulation of memory T cell functions (Kasaian et al., Immunity 16:559-569, 2002.) IL-21, in combination with IL-15 has been shown to induce NK and T cell expression of *interferon* γ (IFN γ), *T-bet*, *IL-2R*α, *IL-12R*β*, IL-18R* and *MyD88* genes (Strengell et al., J.Immunol. 169:3600-3605,2002). IFN γ has been identified as important regulator of innate and adaptive immune responses, promoting Th1 immune responses by activating NK cells, T cells and macrophages (Farrar et al., Annu.Rev. Immunol. 11;571 -611,1993).

IgE plays an important role in the pathogenesis of allergic disorders, such as allergic asthma, allergic rhinitis and atopic dermatitis, which is due to its ability to bind to the high affinity IgER, FcRεI, on mast cells and basophils, thereby triggering immediate-hypersensitivity reactions that result eventually in the release of pharmacological mediators including histamine, leukotrienes, cytokines and chemokines. In addition, following its interaction with CD23, IgE facilitates capture and uptake of allergens by B cells, leading to perpetuation and worsening of allergic conditions, characterized and accompanied by increased IgE serum concentrations (Geha, et al Nat. Rev. Immunol. 3:721, 2003). IgE serum levels are also elevated in parasitic diseases, in which it has a role in host immune defense mechanisms, as well as in some rare genetic disorders of the immune system, including Wiskott Aldrich Syndrome (Derry, et al. Cell 78:635, 1994), Hyper IgE syndrome (Buckley, R. H. Clin. Rev.-Allergy Immunol. 20:139, 2001) and immune dysfunction polyendocrinopathy enteropathy X-linked (IPEX) syndrome (Bennett, et al. Immunopenetics 53:435, 2001). Under non-pathological conditions however, serum IgE levels are much lower than those of other isotypes, indicating that synthesis of IgE is tightly regulated to avoid allergic immune reactions.

The molecular mechanisms underlying the production of IgE are similar to those for other isotypes and are controlled by specific signals from cytokines, as well as the TNFR-superfamily member CD40 (Banchereau, et al. Annu. Rev. Immunol. 12:881, 1994). Isotype switching to IgE involves a two-step process of DNA excision and ligation, followed by a deletional class switch recombination (CSR) event (Manis, et al. Trends Immunol. 23:31, 2002.). CRS results in the deletion of DNA between switch (S) regions, which are located upstream to every constant (C) region gene of the Ig heavy chain, except for δ. A prerequisite for CSR to the production of IgE is the activation of the promoter of the Iε exon, located immediately upstream of the Sε region, which leads to the transcription of an unarranged, germline, ε gene. As the magnitude of germline ε transcription is quantitatively correlated with CSR efficiency (Manis, ibid.), its modulation directly affects the capacity of a B cells to produce IgE (Gauchat, et al. Int. Immunol. 4:397, 1992). The Th2 cytokines IL-4 and IL-13 are potent inducers of Cε germline transcription in human B cells (Gauchat, et al. J. Exp. Med. 172:463, 1990; Punnonen, et al., Proc. Natl. Acad. Sci. USA 90:3730, 1993), functioning through the activation of STAT6 (Nelms, et al., Annu. Rev. Immunol. 17:701,1999).

Recently an association between a single nucleotide polymorphism (T-83C) in the IL-21R and the presence of elevated IgE serum levels has been reported (Hecker, et al., Genes Immun. 3:228, 2003), suggesting that IL-21 has a function in the production of this istoype in humans. The therapeutic effects of IL-21 are being evaluated as a treatment for cancer and other diseases. Therefore, understanding how polymorphisms in the IL-21 receptor effect the biological activities associated with IL-21 administration are useful. The present invention provides methods for predicting and evaluating patient response to IL-21 based on IL-21 receptor genotype. This use, as well as other uses, should be apparent to those skilled in the art from the teachings herein.

### SUMMARY OF THE INVENTION

In one aspect, the present invention provides a method of predicting therapeutic response to IL-21 in an individual in need of IL-21 therapy comprising obtaining a biological sample from the patient and detecting the genotype of the individual's *IL-21* receptor gene. In one embodiment, the genotype of the individual's *IL-21* receptor gene comprises one allelic variant. In another embodiment, the genotype of the individual's *IL-21* receptor gene comprises two allelic variants. In another embodiment, the genotype of the individual's *IL-21* receptor gene comprises an IL-21 haplotype. In further embodiments, the allelic variant is a T-83C variant.

In another aspect, the present invention provides a method of selecting a therapeutic regimen for use of IL-21 comprising obtaining a biological sample from an individual; detecting the genotype of the individual's *IL-21* receptor gene; and determining a therapeutically effective dose of an IL-21 composition wherein the IL-21 dose differs from the dose given to individuals that are homozygous for the wild-type *IL-21* receptor gene. In one embodiment, the therapeutically effective dose is an optimal immunological dose. In another embodiment, the genotype of the individual's *IL-21* receptor gene comprises two allelic variants. In another embodiment, the genotype of the individual's *IL-21* receptor gene comprises one allelic variant. In further embodiments, the allelic variant is a T-83C variant. Additional embodiments include administering the individual a therapeutically effective dose. Further embodiments include wherein the genotype of the individuals *IL-21* receptor gene comprises an *IL-21* haplotype.

### BRIEF DESCRIPTION OF THE FIGURES

Figures 1A and 1B- Addition of IL-4 to CD19+ peripheral B cells induced production of IgE.

Figure 1C-IL-21 enhanced IL-4-induced IgE production in CD19+CD27- naïve B cells.

Figure 2A- Stimulation of purified B cells with the combination of anti-CD40 mAb and IL-4 resulted in a strong induction of germline Cε transcription.

Figure 2B-IL-21 did not affects IL-4-induced gerraline Ce transcription in human spleen or periphery blood-derived B cells.

Figure 3A-IL-21 inhibited the production of IgE in cultures of PBMC at concentration as low as 3 ng/ml, in ten out of 15 healthy donors tested.

Figure 3B. In five donors, the inhibitory effects of IL-21 on IgE synthesis were only detected at concentration ≥30 ng/ml, whereas IL-21 enhanced IL-4-induced IgE production between 1-10 ng/ml.

Figures 4A and 4B. In PBMC cultures stimulated with IL-4 and IL-21, levels of IL-4-induced IgE production inversely correlated to IFN-γ in sensitive groups of donors.

Figures 4C and 4D- In PBMC cultures stimulated with IL-4 and IL-21, levels of IL-4-induced IgE production inversely correlated to IFN-γ in insensitive groups of donors.

Figures 4E and 4F addition of IL-21 to cultures of PBMC stimulated with IL-4 in the presence of an anti-CD40 mAb resulted in a strong enhancement of IgE production.

Figure 5 the addition of either anti-IFN-γR1 antibody or non-relevant isotype matched control antibody at the onset of cultures alone did not affect IL-21-mediated inhibition of IgE production.

Figure 6A levels of IL-4-induced IgE production in vitro did not differ significantly between the healthy and allergic donors.

Figure 6B IL-21-induced modulatory effects on IL-4-induced IgE production did not differ between healthy and allergic donors.

Figure 7A-Both donors who were homozygous or heterozygous for the wild-type IL-21R allele showed differential IL-4 induced IgE synthesis *in vitro.*

Figure 7B-Differential IL-4-induced IgE synthesis mediated by IL-21 was shown in for donors that were homozygous and heterozygous for the IL-21R wildtype allele.

Figure 7C-IL-21 induced higher levels of IFN-γ production by PBMC from wildtype individuals as compared to those the IL-21R polymorphic variant.

### DESCRIPTION OF THE INVENTION

Prior to setting forth the invention in detail, it may be helpful to the understanding thereof to define the following terms:

The term "allelic variant" is used herein to denote any of two or more alternative forms of a gene occupying the same chromosomal locus. Allelic variation arises naturally through mutation, and may result in phenotypic polymorphism within populations. Gene mutations can be silent (no change in the encoded polypeptide) or may encode polypeptides having altered amino acid sequence. The term allelic variant is also used herein to denote a protein encoded by an allelic variant of a gene.

The terms "amino-terminal" and "carboxyl-terminal" are used herein to denote positions within polypeptides. Where the context allows, these terms are used with reference to a particular sequence or portion of a polypeptide to denote proximity or relative position. For example, a certain sequence positioned carboxyl-terminal to a reference sequence within a polypeptide is located proximal to the carboxyl terminus of the reference sequence, but is not necessarily at the carboxyl terminus of the complete polypeptide.

The term "cancer" or "cancer cell" is used herein to denote a tissue or cell found in a neoplasm which possesses characteristics which differentiate it from normal tissue or tissue cells. Among such characteristics include but are not limited to: degree of anaplasia, irregularity in shape, indistinctness of cell outline, nuclear size, changes in structure of nucleus or cytoplasm, other phenotypic changes, presence of cellular proteins indicative of a cancerous or pre-cancerous state, increased number of mitoses, and ability to metastasize. Words pertaining to "cancer" include carcinoma, sarcoma, tumor, epithelioma, leukemia, lymphoma, polyp, and scirrus, transformation, neoplasm, and the like.

The term "co-admininistration" is used herein to denote that an IL-21 polypeptide or protein and at least one other therapeutic agent may be given concurrently or at different times of a treatment cycle. The co-adminstration may be a single co-adininistration of both IL-21 and the other therapeutic agent or multiple cycles of co-administration, where both IL-21 and and the other therapeutic agent are both given, at least once, within a three month period. Co-administration need not be the only times either IL-21 or and the other therapeutic agent is administered to a patient and either agent may be administered alone or in a combination with therapeutic agents other than IL-21.

The term "combination therapy" is used herein to denote that a subject is administered at least one therapeutically effective dose of an IL-21 composition ("IL-21") and at least one additional therapeutic agent. The IL-21 composition may be a mature polypeptide, fragment thereof, fusion or conjugate that demonstrates IL-21 biological activity.

The term "genotype" refers to the specific allelic composition of an entire cell or a certain gene, whereas the term "phenotype" refers to the appearance or other outward manifestations of a specific genotype.

The term "haplotype" denotes a sequence of nucleotides found at one or more polymorphic sites in a locus on a single chromosome from a single individual. The haplotype may be a sequence of nucleotides found at all or a subset of polymorphic sites in a locus on a single chromosome from a single individual, resulting in a "full haplotype" or a "partial haplotype".

The term "heterozygous" refers to individuals that carry two allelic variants at a specific polymorphic site. The term "homozygous" refers to individuals that carry two copies of the same allele, and may include the wild-type allele or allelic variants.

The term "isolated", when applied to a polynucleotide, denotes that the polynucleotide has been removed from its natural genetic milieu and is thus free of other extraneous or unwanted coding sequences, and is in a form suitable for use within genetically engineered protein production systems. Such isolated molecules are those that are separated from their natural environment and include cDNA, genomic clones and polymerase chain reaction (PCR) amplicons. Isolated DNA molecules of the present invention are free of other genes with which they are ordinarily associated, but may include naturally occurring 5' and 3' untranslated regions such as promoters and terminators. The identification of associated regions will be evident to one of ordinary skill in the art (see for example, Dynan and Tijan, Nature 316:774-78, 1985).

An "isolated" polypeptide or protein is a polypeptide or protein that is found in a condition other than its native environment, such as apart from blood and animal tissue. In a preferred form, the isolated polypeptide is substantially free of other polypeptides, particularly other polypeptides of animal origin. It is preferred to provide the polypeptides in a highly purified form, i.e. greater than 95% pure, more preferably, greater than 99% pure. When used in this context, the term "isolated" does not exclude the presence of the same polypeptide in alternative physical forms, such as dimers or alternatively glycosylated or derivatized forms.

The term "level" when referring to immune cells, such as NK cells, T cells, in particular cytotoxic T cells, B cells and the like, an increased level is either increased number of cells or enhanced activity of cell function.

The term "level" when referring to viral infections refers to a change in the level of viral infection and includes, but is not limited to, a change in the level of CTLs or NK cells (as described above), a decrease in viral load, an increase antiviral antibody titer, decrease in serological levels of alanine aminotransferase, or improvement as determined by histological examination of a target tissue or organ. Determination of whether these changes in level are significant differences or changes is well within the skill of one in the art.

The term "neoplastic", when referring to cells, indicates cells undergoing new and abnormal proliferation, particularly in a tissue where in the proliferation is uncontrolled and progressive, resulting in a neoplasm. The neoplastic cells can be either malignant, i.e. invasive and metastatic, or benign.

The term "optimal immunological response" refers to a change in an immunological response after administration of IL-21, and can be (1) an increase in the numbers of activated or tumor specific CD8 T cells, (2) an increase in the numbers of activated or tumor specific CD8 T cells expressing higher levels of granzyme B or perforin or IFNγ, (3) upregulation of Fcy receptor (CD16, CD32, or CD64) on Nk cells, monocytes, or neutrophils, (4) an increase in soluble CD25 in the serum, (5) reduction in serum level of proteins liberated by tumor cells (see, Taro et al., J. Cell Physiol. 203(1):1-5, 2005), for example, carcinoembryonic antigen (CEA), IgG, CA-19-9, or ovarian cancer antigen (CA125), (6) an increase in the numbers of NK cells expressing higher levels of granzyme B, perforin or IFNγ, (7) increase in the levels of activation cytokines such as IL-18, IL-15, IFNγ and chemokines that enable homing of effector cells to the tumor, such as IP-10, RANTES, IL-8, MIP1a or MIP1b, (8) an increase in the numbers of activated macrophages in the periphery or at the tumor site, where activation can be detected by expression of increased MHC class I or Class II, production of IL-15, IL-18, IFNγ, or IL-21, or (9) macrophage activity as indicated by decline in red blood cell count (severity of anemia). The IL-21 dose that produces the optimal immunological response is the "optimal immunological dose."

The term "polymorphism" refers to the coexistence of at least two alternative sequences in a gene or portion thereof. The difference in the sequences can be a single nucleotide and can encompass substitutions, insertions, or deletions, and may or may not result in detectable differences in gene expression or protein function.

The term "receptor" denotes a cell-associated protein that binds to a bioactive molecule (i.e., a ligand) and mediates the effect of the ligand on the cell. Membrane-bound receptors are characterized by a multi-peptide structure comprising an extracellular ligand-binding domain and an intracellular effector domain that is typically involved in signal transduction. Binding of ligand to receptor results in a conformational change in the receptor that causes an interaction between the elector domain and other molecule(s) in the cell. This interaction in turn leads to an alteration in the metabolism of the cell. Metabolic events that are linked to receptor-ligand interactions include gene transcription, phosphorylation, dephosphorylation, increases in cyclic AMP production, mobilization of cellular calcium, mobilization of membrane lipids, cell adhesion, hydrolysis of inositol lipids and hydrolysis of phospholipids, and induction of proliferation, differentiation or apoptosis. In general, receptors can be membrane bound, cytosolic or nuclear; monomeric (e.g., thyroid stimulating hormone receptor, beta-adrenergic receptor) or multimeric (e.g., PDGF receptor, growth hormone receptor, IL-3 receptor, GM-CSF receptor, G-CSF receptor, erythropoietin receptor and IL-6 receptor).

The term "therapeutically effective amount" is defined as an amount of an IL-21 composition or IL-21 composition in combination additional therapeutic compositions that results in a complete response, partial response, or stable disease with an increased time to progression over the median response duration for therapy without IL-21.

The term "tumor associated antigen" refers a peptide or polypeptide or peptide complex that has a different expression profile from antigen found on a non-tumor cells. For example, a non-tumor antigen may be expressed in higher frequency or density by tumor cells than by tumor cells. A tumor antigen may differ from a non-tumor antigen structurally, for example, the antigen could be expressed as a truncated polypeptide, have some mutation in the amino acid sequence or polynucleotide sequence encoding the antigen, be misfolded, or improperly modified post-translationally. Tumor antigens are similar to antigens that are present on normal, non-tumor cells in the host organism and thus allow the tumor cells to escape the host's immunological surveillance mechanisms.

The term "wildtype" denotes an allele that functions normally and represents a common type found in natural populations at a frequency of at least one percent.

Molecular weights and lengths of polymers determined by imprecise analytical methods (e.g., gel electrophoresis) will be understood to be approximate values. When such a value is expressed as "about" X or "approximately" X, the stated value of X will be understood to be accurate to ± 10%.

All references cited herein are incorporated by reference in their entirety.

The present invention is based in part upon the discovery that a polymorphism in the IL-21 receptor (IL-21R) is associated with reduced IL-21 induction of interferes γ (IFN-γ).

### A. Description of IL-21.

Human IL-21 is described in commonly-owned U.S. Patent Nos. 6,307,024, and 6,686,178, WO 04/055168, and Parrish-Novak et al. (Nature 408:57-63, 2000), all of which are incorporated herein by reference. The IL-2 receptor is described in commonly-owned U.S. Patent Nos. 6,576,744 and 6,803,451, which are incorporated herein by reference, and U.S. Patent 6,057,198. As described in these publications, the sequence of the human *IL21* cDNA contains an open reading frame that encodes a polypeptide precursor of 162 amino acids. The mature polypeptide has a predicted molecular weight of 15 Kd, and consists of a 131 amino acid 4-helix-bundle cytokine domain with highest sequence and structural homology to IL-15 and IL-2. IL-21 is produced by CD4+ activated T cells and stimulates immune elector cells. IL-21 has been shown to regulate B cell, T cell and NK cell activation and, expansion. IL-21 has been shown to have potent antitumor activity in vivo (Wang et al., Cancer Res. 63:9016-9022, 2003 and Brady et al., J. Immunol. 172:2048-2058,2004.)

### B. Polymorphisms of the IL-21 receptor.

The IL-21R belongs to the Class I cytokine receptor subfamily that includes, but is not limited to, the receptors for IL-2, IL-4, IL-7, IL-15, EPO, TPO, GM-CSF and G-CSF (for a review see, Cosman, "The Hematopoietin Receptor Superfamily" in Cytokine 5(2): 95-106, 1993). The IL-21 receptor has been identified on NK cells, T cells and B cell indicating IL-21 acts on hematopoietic lineage cells. Other known four-helical-bundle cytokines that act on lymphoid cells include IL-2, IL-4, IL-7, and IL-15. For a review of four-helical-bundle cytokines, see, Nicola et al., Advances in Protein Chemistry 52:1-65, 1999 and Kelso, A., Immunol. Cell Biol. 76:300-317,1998.

The *IL21R* gene is located on human chromosome 16p11, near *IL4RA*. Comparison of our *IL21R* cDNA sequence with the genomic sequence (AC002303) revealed that the gene is organized into 9 exons, spans about 20 kilobases (kb) of genomic DNA, and lies 65 kb from the *IL4RA* gene. Sequencing of 12 Mb of the chromosomal region containing the *IL21R* gene revealed the presence of multiple copies of large duplicated segments originating in other regions of the genome (Loftus et al., Genomics 60:295-308, 1999). These duplicated segments, or duplicons, are interspersed throughout the pericentromeric regions of several chromosomes, and can predispose their target regions to additional duplications or deletions (Eichler et al., Genome Res. 8:758-762, 1998). The 16p11 region appears to be one of the most prone to accumulate these duplicated segments, as more than half of the identified segments are represented at least once (Eichler ibid.). It is unclear whether these repeats mediate chromosomal abnormalities that disrupt the *IL21R* locus.

The methods of the present invention are based on the discovery that individuals respond with a differential sensitivity to the modulatory effects of IL-21 on induction of IFN-γ found to be associated with the presence of at least one polymorphism in the gene encoding the IL-21R. In one embodiment, the polymorphism is the consequence of a nucleotide substitution in the 5' flanking region of exon 1B (Heckler et al., Genes Immun. 3:228-233, 2003). The presence of the T-83C variant was found to be associated with enhanced IgE serum levels in healthy, and to a greater extent in atopic, individuals (Heckler ibid.). As described herein, T or NK cells from individuals who carry one allele of this IL-21R gene variant produce less IFN-γ following stimulation with IL-21, as compared to those from individuals carrying the wild-type gene. Accordingly, B cells from the individuals with the variant allele require higher amounts of IL-21 to achieve a comparable degree of IFN-γ-mediated inhibition of IgE production. Moreover, IgE production levels, at lower concentrations of IL-21 seem to be more prominent in the IL-21R gene variant group. It is therefore possible that due to its strong growth-promoting effects on Ig-committed B cells (Parrish-Novak et al. (Nature 408:57-63, 2000), IL-21 also differentially affects the proliferation of B cells in relation to the polymorphism of its receptor expressed on cells. However, the interaction between the IL-21R and IL-21 on B cells expressing the variant IL-21R is the subject of further investigation.

In both groups of donors IL-21 enhanced IgE production was at the lowes concentrations used, and in contrast it seems that relatively higher concentrations of IL-21 are necessary to stimulate IFN-γ production by T or NK cells. While not intending to be bound by theory one explanation for this finding is that the levels of the IL-21R differ among the cell lineages on which it is expressed. Support for this notion comes from a recent study in the mouse in which it was shown that expression levels of the IL-21R are consistently higher on activated B cells, as compared to activated T and NK cells (Jin, et al. J Immunol 173:657-665. 2004.) Likewise, the decrease in IL-21-mediated production of IFN-γ from individuals carrying the T-83C variant of the IL-21R could also be due to lower expression levels of this receptor by T or NK cells as compared to individuals homozygous for the wild-type IL-21R gene. The decrease in IL-21-induced production of IFN-γ from individuals carrying the T-83C variant of the IL-21R may be due to altered expression levels of this receptor by T or NK cells as compared to individuals homozygous for the wild-type IL-21R gene. Alternatively, the presence of the T-83C allele may result in the expression of a receptor with a lower affinity for its ligand.

Individuals with either one or two variant IL-21R alleles can be identified (Heckler et al., ibid, 2003). It has been reported that individuals homozygous for the T-83C variant gene had higher serum levels of IgE, as compared to heterozygous individuals, suggesting a gene dose effect. The previously reported association between the genetic variation of the IL-21R and the presence of elevated serum IgE levels was in females only (Heckler et al., ibid.), however, a possible gender-restricted modulatory effect of IL-21 on the synthesis of IgE is not supported by the discovery as disclosed herein.

### C. IL-21 receptor genotype and IL-21 therapy

It is well established that heterogeneity in efficacy and toxicity of drugs is observed in patient populations. It is generally accepted that genetic differences impact treatment outcome in patients, including genetic variation in genes encoding drug targets such as receptors (Evans, W.E and Relling, M.V., Science 286:487-491, 1999; Evans, W. E and McLeod, H.L., N. Engl. J. of Med. 348:538-549, 2003). Customized therapy based on identity of a particular allele in an individual is known as pharmacogenomics. Information obtained using known diagnostic assays identifying IL-21 receptor variants can guide a clinician in recommending a therapeutic regimen for treating IL-21 responsive diseases, such as cancer, infection and autoimmune disorders.

In one aspect, the present invention provides diagnostic methods for predicting therapeutic response to IL-21 in an individual in need of IL-21 therapy. In particular, the present invention provides diagnostic methods for a patient contemplating use of IL-21 as an immunotherapeutic treatment for cancer, infection or autoimmune disorders. The method comprises obtaining a patient biological sample and detecting the genotype of an individual's IL-21 receptor gene. Based on the present invention detection of the IL-21 receptor genotype will be correlated to an optimal immunological dose of an IL-21 composition leading to a positive therapeutic outcome. Methods of determining an individual's IL-21 receptor genotype or polymorphism comprise screening a biological sample as such as a suitable cell or tissue sample isolated from the individual. In certain embodiments of the present invention the genotype of the individual's IL-21 receptor gene comprises one allelic variant. In one embodiment, the genotype of the individual's IL-21 receptor gene comprises two allelic variants. Another embodiment of the present invention provides methods for predicting therapeutic response wherein an IL-21 haplotype relates to the T-83C allelic variant. In another embodiment, the allelic variant will be the T-83C allelic variation.

In another aspect, the present invention provides a method for selecting a therapeutic regimen for use of IL-21. In certain embodiments, the therapeutic regimen is for use of IL-21 in the treatment of cancer, infection or autoimmune disorders in the individual. The selection of the therapeutic regimen for use of IL-21 comprises obtaining a biological sample from an individual; detecting the genotype of individual's IL-21 receptor gene and determining a therapeutically effective dose of an IL-21 composition, wherein the dose of IL-21 differs from the dose given to individuals that are homozygous for the wild-type IL-21 receptor gene. The method further comprises administering to the individual a therapeutically effective dose of an IL-21 composition which differs from the dose given to individuals where the allelic variation in the IL-21 receptor has not been identified. In certain embodiments of the present invention the genotype of the individual's IL-21 receptor gene comprises one allelic variant. In another embodiment, the genotype of the individual's IL-21 receptor gene comprises two allelic variants. In another embodiment, the allelic variant will be the T-83C allelic variation.

Positive therapeutic outcome can be measured using objective status protocols to assess solid tumor response. Representative criteria include the following: (1) Complete Response (CR) defined as complete disappearance of all measurable and evaluable disease with no new lesions, and no disease related symptoms. No evidence of non-evaluable disease; (2) Partial Response (PR) defined as greater than or equal to 30% decrease from baseline in the sum of products of perpendicular diameters of all measurable lesions, with no progression of evaluable disease, no new lesions. According the RESIST criteria, patients with at least one measurable lesion; (3) Progression defined as 20% or an increase of 10 cm2 in the sum of products of measurable lesions over the smallest sum observed using same techniques as baseline, or clear worsening of any evaluable disease, or reappearance of any lesion which had disappeared, or appearance of any new lesion, or failure to return for evaluation due to death or deteriorating condition (unless unrelated to this cancer); (4) Stable or No Response defined as not qualifying for CR, PR, or Progression. (See, Clinical Research Associates Manual, Southwest Oncology Group, CRAB, Seattle, WA, Oct. 6, 1998, updated Aug.1999).

The detection of IL-21 receptor genotype can use methods known in art, including, but not limited to, testing blood cells or DNA from the individual to determine if a mutation in the DNA sequence is present. In certain embodiments, the T to C mutation in the DNA sequence is present at position -83. Position 1 of the human IL-21 receptor is defined as the first nucleotide of exon 1b and corresponds to position 36058 of human chromosome 16 BAC clone AC004525 (Heckler et al., Genes and Immunity 4:228-233, 2003.) Heterozygous individuals carry one copy of the T-83C allele of the IL-21 receptor and homozygous individuals carry two copies of the T-83C allele. Both heterozygous and homozygous individuals may have reduced ability to elicit a positive therapeutic response when administered an IL-21 dose used for individuals that do not carry a copy of the mutated allele.

The present invention also provides a method of predicting therapeutic response to IL-21 in an individual in need of IL-21 therapy comprising obtaining a biological sample from the patient and detecting the genotype where the genotype of the individual's IL-21 receptor gene comprises an M-21 haplotype. The present invention additionally provides a method of selecting a therapeutic regimen for use of IL-21 comprising obtaining a biological sample from an individual; detecting the genotype where the genotype of the individual's IL-21 receptor gene comprises an IL-21 haplotype; and determining a therapeutically effective dose of an IL-21 composition wherein the IL-21 dose differs from the dose given to individuals that are homozygous for the wild-type IL-21 receptor gene.

Sequencing techniques are known in the art. For example, allelic variants and mutations can be directly sequenced by comparing a sample sequence to a corresponding wild-type sequence. Known techniques include, but are limited to, Maxam and Gilbert (Proc. Natl. Acad. USA 74:560, 1977), Sanger et al. (Proc. Natl. Acad. USA 74:5463, 1977), U.S. Patent No. 5,547,835, U.S. Patent No. 5,605,798, Cohen et al. (Adv. Chromat. 36:127-162, 1996), Griffin et al. (Appl. Biochem. Bio, 38; 147-159,1993), U.S. Patent No. 5,580,732, U.S. Patent No. 5,571676.

Additional methods for genotyping analysis include PCR-based amplification of the selected region, in the presence of allele-specific fluorescent probes; melting curve analysis of PCR amplicons in the presence of DNA-intercalating fluorescent dyes or labeled, allele-specific probes; hybridization of amplified DNA to sequence-specific or allele-specific oligonucleotides on DNA micro-arrays; detection of DNA fragments by mass-spectrometry or LC/MS/MS; altered susceptibility to digestion by restriction enzymes (restriction fragment length polymorphism); altered mobility of single-stranded DNA on polyacrylamide gels (single-strand conformational polymorphism); and other techniques known to those skilled in the art. (See, e.g., Daly, Ann K. Arch. Pharmacol., 369:133-140, 2004.)

Another method for detecting the IL-21 receptor genotype utilizes antibodies that can distinguish between allelic forms of the receptor by binding certain forms of the receptor with specificity or determine relative levels of expression on hematopoietic cells. White blood cells (WBC) or subsets of WBC are isolated from an individual using techniques that are well known to those skilled in the art. Diagnostic procedures cn also be performed in situ on tissue sections obtained from patients obtained from biopsies or resections. Measurement of antibody binding to cells can use any known method, for example, quantitative flow cytometry, enzyme-linked immunoassay or fluorescence-linked immunoassay. Such measuring techniques can identify a phenotype associated with the allelic variation. This phenotype may include, but is not limited to, altered protein conformation leading to distinct epitopes, altered protein expression or alterations in the normal binding partners of the IL-21 receptor protein.

The invention is further illustrated by the following non-limiting examples.

### EXAMPLES

### Example 1

### A. Donors and cells

Human PBMC were isolated from 15 healthy donors and 17 atopic patients (Service des Maladies Respiratoires, CHU Arnaud de Villeneuve, Montpellier, France) by centrifugation over Ficoll-Hypaque (Amersham Biosciences, Uppsala, Sweden). Atopy was defined on the basis of elevated, allergen-specific aerum IgE levels, a positive skin prick test and a positive clinical score, as a result of exposure to common aeroallergens. Highly purified (purity > 98%) CD19+ spleen B cells (Service de Chirurgie Digestive, CHU St Eloi, Montpellier, France) were obtained from human spleen fragments of healthy organ donors by positive selection using specific mAb-coated magnetic beads (Miltenyi, Bergisch Gladbach, Germany) and a preparative magnetic cell sorter (Miltenyi), according to the according to the manufacturer's recommendations. Purified CD19+ B cells (purity > 98%) were also isolated from PBMC (Etablissement Français du Sang, Montpellier) using the Rosettesep® procedure (StemCell Technologies, Meylan, France), according to the manufacturer's recommendations. Naïve B cells were obtained following staining of CD19+ B cells with a FITC-conjugated anti-CD27 mAb (PharMingen, La Jolla, CA) and sorting of CD19+,CD27- B cells, using a FACS Vantage® (Becton Dickinson, San Jose, CA), according the procedure described in the literature (Scheffold et al. 2002. Methods in Microbiology Vol 32, ed. S. H. E. Kaufmann, and D. Kabelitz, eds. Academic Press, San Diego, CA. pp707-749). Re-analysis of sorted cells showed >99% purify.

### B. Reagents and culture conditions

Induction of Ig production was carried out as follows: naïve or memory CD19+ human B lymphocytes (106/ml) were cultured with 10⁶g/ml of the anti-CD40 mAb 89 (Valle et al., Eur. J. Immunol, 19:1493, 1989), in the presence or absence of combinations of recombinant (r)IL-4 (Schering-Plough, Dardilly, France) and rIL-21 (ZymoGenetics, Seattle, WA) in flat-bottom 96-well culture plates (Nunc, Roskilde, Denmark) in Yssel's medium (Yssel et al., J. Immunol. Methods 72:219,1984), supplemented with 10% FCS in sextuplet in a final volume of 200 µl. PBMC (106/ml) were cultured with the same combinations of cytokines, in the presence or the absence of the anti-CD40 mAb. Where indicated, a neutralizing polyclonal goat anti-human IFN-γ receptor 1 (anti-IFN-γ R1) antibody (R&D Systems Europe, Lille, France), an anti-IFN-γ R1 mouse mAb (Becton Dickinson) or a normal purified mouse IgG1 (Southern Technologies, Birmingham, A1) was added at a concentration of 2 µg/ml at the initiation of the cultures and subsequently at days 2 and 4, at the same concentration. After 12 days of incubation at 37° and 5% CO2, culture supernatants were collected and IgE content was measured. The production of IFN-γ was determined on the same culture supernatants collected at day 12 in parallel cultures. For analysis of IgE transcripts, purified CD19+ B cells were cultured under the same experimental conditions and cells were harvested for mRNA isolation after 5 days of culture.

### C. Measurement of IgE and IFN-γ production

IgE and IFN-γ content of culture supernatants was determined by isotype- and cytokine-specific ELISA, respectively, as described in Lecart et al. (Int. Immunol. 14:1351, 2002) and Lecart et al. (J. Invest. Dermatol. 117 :318,2001).

cDNA synthesis, RT-PCR analysis, and Northern blotting analysis of germline Ig transcripts RNA extraction, reverse transcription and amplification of cDNA (corresponding to 1µg of RNA per PCR sample) was carried out as described Lecart et al. (Int. Immunol. 14:1351, 2002). The nucleotide sequences of PCR primers, the Cε germ-line and β-actin transcripts were as follows:

Cε germline
Sense Iε5' GAC GGG CCA CAC CAT CC (SEQ ID NO: 1)
Antisense Cε5' CGG AAG GTG GCA TTG GAG G (SEQ ID NO: 2)

β-actin
sense β-actin: 5' GCT GCT GAC CGA GGC CCC CCT GAA C (SEQ ID NO: 3)
antisense β-actin: 5' CTC CTT AAT GTC ACG CAC GAT TTC (SEQ ID NO: 4)

Germline ε and β-actin mRNA transcripts were analyzed by Northern Blotting using cRNA probes, complementary to Cε and β-actin mRNA, as described in Gauchat et al. (J. Exp. Med. 172:463, 1990).

### D. Genotyping of the IL-21R gene

Genomic DNA was isolated from peripheral blood by standard phenol-chloroform extraction followed by ethanol precipitation (Blin, N., and D. W. Stafford. 1976. Nucleic Acids Res. 3:2303-2308). A 521-bp PCR fragment including the -83T>C polymorphism was amplified using the primers: 5'-GCC TGC TGC ATC TAG TGT C-3' (upstream; SEQ ID NO: 5) and 5'-CCG TGC TTC ATG AGA AAG A-3' (downstream; SEQ ID NO: 6). PCR were carried out in a total volume of 50 µl containing 100 ng DNA, 0.5 µM of each primer, 0.2 mM of each dNTP, 1.5 mM MgC12, 5 µl 10x Buffer, and I U Taq DNA polymerase (Invitrogen). Samples were denatured at 94°C for 10 min followed by 32 cycles at 94°C for 45 s, 55°C for 30 s, 72°C for 60 s, and a final 10 min extension at 72°C. The fragments were sequenced using the ABI Prism Big Dye Terminator DNA Sequencing Kit (PE Applied Biosystems, Foster City, CA, USA) according to the manufacturers instructions, and analysed on an ABI Prism 310 Genetic Analyser (Perkin-Elmer, Boston. MA). The IL21R sequencing primer was: 5'-CGT GCT TCA TGA GAA AGA TG-3' (SEQ ID NO: 7).

### E. IL-21 enhances IgE production by human CD19+ B cells stimulated with anti-CD40 in the presence of IL-4.

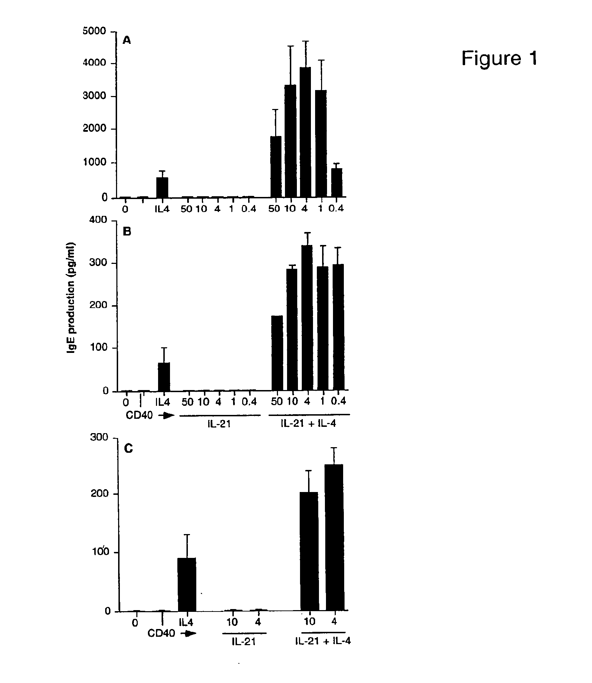

The capacity of rIL-21 to modulate the production of rIL-4-induced IgE production was investigated using purified human CD19+ peripheral blood- or spleen-derived B cells, obtained from healthy non-atopic donors, that were stimulated with anti-CD40 mAb in the presence of rIL-4 and increasing concentrations of rIL-21. As expected, addition of rIL-4 to cultures of these cells induced the production of IgE (Fig.1A and IB). rIL-21 alone did not induce IgE synthesis but strongly enhanced IL-4-induced IgE production, in a dose-dependent fashion, irrespective of the origin of the B cells. In addition, rIL-21 enhanced rIL-4-induced IgE production in purified splenic CD19+,CD27-naïve B cells (Fig. 1C). Optimal IgE production-enhancing capacity of rIL-21 was observed at concentrations between 1-10 ng/ml of cytokine.

### F. IL-21 does not affect IL-4-induced expression of germline Cε transcripts in purified B cells

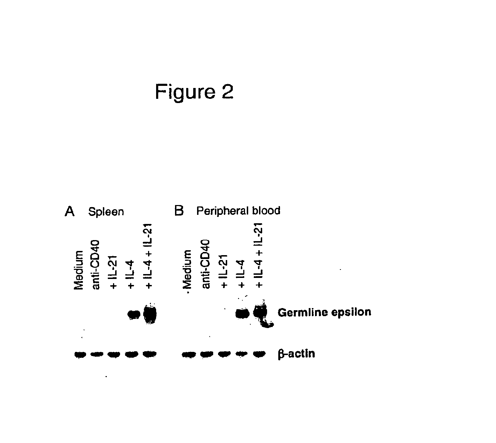

The expression of Cε-specific germline transcripts is considered to be an obligatory event, preceding successful isotype switching of both mouse and human B cells to the production of IgE. To investigate whether IL-21-enhanced IgE production was associated with an increase in IL-4-induced isotype switching, the expression of germline ε RNA by anti-CD40 mAb and IL-4-stimulated CD19+ B cells was determined by Northern blotting analysis. In agreement with results reported in the literature, stimulation of purified B cells with the combination of anti-CD40 mAb and rIL-4 resulted in a strong induction of germline Cε transcription (Fig. 2A), whereas stimulation via CD40 alone did not have any effect. Expression of these transcripts was not observed in cells stimulated with rIL21 and anti-CD40 mAb. In addition, rIL-21 did not affect rIL-4-induced germline Cε transcription, neither in human spleen- nor in peripheral blood-derived B-cells (Fig. 2B). These results indicate that the enhancement of IL-4-induced IgE synthesis by IL-21 is not due to an increase in germline Cε RNA synthesis, but is likely to be the consequence of its capacity to promote expansion of activated B cells.

### Example 2

### IL-21 indirectly inhibits IL-4-induced IgE production via the induction of IFN-γ production by T and/or NK cells

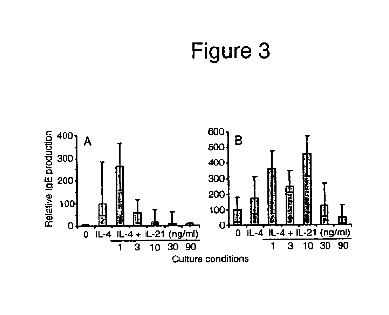

IL-21 has been shown to enhance IFN-γ production by human T and NK cells (Strengell et al., J. Immunol. 170:5464, 2003). Furthermore, IFN-γ is known to strongly inhibit IL-4-induced IgE production by B cells (Pène et al., Proc. Natl. Acad. Sci. 85:6880, 1988), suggesting that IL-21 might have an inhibitory role on IgE synthesis through the enhancement of production of this cytokine. This possibility was investigated by comparing the effect of rIL-21 on rIL-4-induced IgE production between populations of B cells that either contained, or were devoid of, T and NK cells. Addition of rIL-4 to cultures of PBMC, in which the latter lymphocytes were present, resulted in the production of low, but reproducible, levels of IgE. In contrast to its effect on purified CD19+ peripheral B cells (Fig. 1), rIL-21 inhibited the production of IgE in cultures of PBMC, at concentrations as low as 3 ng/ml, in ten out of fifteen healthy donors tested (Fig. 3A). However, at the lowest concentration of IL-21 tested, IL-4-induced IgE production by B cells of these donors was significantly and reproducibly enhanced. In the five other donors, the inhibitory effects of rIL-21 on IgE synthesis were only detected at concentrations equal or higher than 30 ng/ml, whereas rIL-21 enhanced IL-4-induced IgE production at concentrations ranging between 1 and 10 ng/ml (Fig. 3B). These results suggest the existence of a differential sensitivity of these two groups of donors to respond to the inhibitory effects of IL-21.

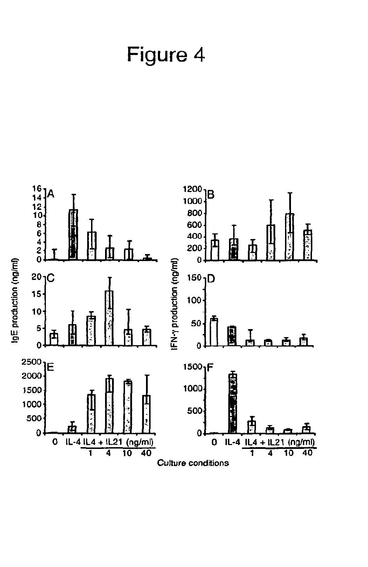

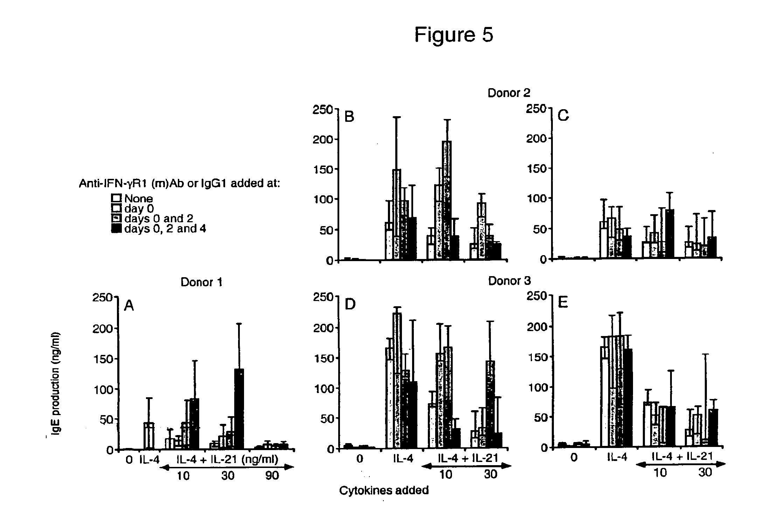

In order to determine whether the IL-21-induced inhibitory effects could be explained by its capacity of induce the synthesis of IFN-γ, production levels of the latter cytokine were measured in cultures of PBMC stimulated with rIL-4 and rIL-21. Levels of rIL-4-induced IgE production were found to inversely correlate with those of IFN-γ, detected in these cultures, both in the sensitive (Fig. 4A and 4B) and the less sensitive (Fig. 4C and 4D) group of donors. It is of note that addition of rIL-2 to cultures of PBMC, stimulated with rIL-4 in the presence of an anti-CD40 mAb, resulted in a strong enhancement of IgE production (Fig. 4E and 4F), reminiscent of that observed in cultures of purified B cells. Under this experimental condition, B cells undergo strong polyclonal CD40-mediated stimulation and the inhibitory effects of IFN-γ on IL-4-induced IgE synthesis are therefore completely masked by the B cells growth-promoting effects of lL-21. To directly demonstrate that IL-21 inhibits the production of IgE, via the induction of IFN-γ production by non-B cells, either a neutralizing goat-anti-IFN-γ R1 antibody or a mouse-anti-IFN-γ R1 mAb was added, at various time points, to cultures of PBMC stimulated with both rIL-4 and various amounts of rIL-21. The addition of either anti-IFN-γR1 antibody at the onset of the cultures alone did not affect the IL-21-mediated inhibition of IgE production, (Fig. 5). However, their repeated addition during the first four days of culture resulted in a complete neutralization of IFN-γ production, thereby restoring the production of IgE to levels comparable, or even superior, to those observed with rIL-4 alone. The addition of a non-relevant isotype matched control antibody had no effect (Fig. 5). At a concentration of 90 ng/ml, IL-21 completely inhibited IL-4-induced IgE synthesis, irrespective of the presence of the polyclonal goat-anti-IFN-γRI antibody, indicating that under these conditions the amount of IFN-γ induced by IL-21 could no longer be neutralized.

### Example 3

### The magnitude of inhibition of IL-4-induced IgE synthesis by IL-21 is determined by a genetic polymorphism of the IL-21R

It has been reported that the presence of a polymorphism (T-83C) in the gene encoding the human IL-21R is associated with elevated serum levels of IgE, in both healthy and, to a greater extent, atopic individuals (Heckler et al. ibid.). Because of the differential sensitivity of PBMC to respond to the effects of rIL-21, as described above, we therefore determined, in a sizeable cohort of donors consisting of both healthy and atopic individuals, whether their clinical status, as well as the presence of this polymorphism, might affect the capacity of IL-21 to modulate IL-4-induced IgE synthesis *in vitro*.

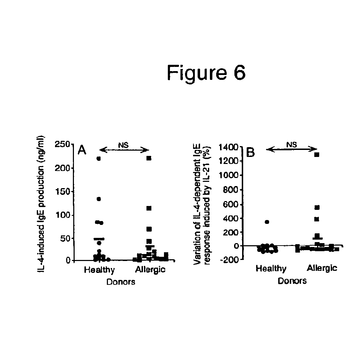

In a first series of experiments investigated whether PBMC of all healthy and allergic donors included in the study differed in their capacity to respond to the IgE production-inducing effects of IL-4. Levels of rIL-4-induced IgE production *in vitro* did not differ significantly (p=0.56, Mann-Whitney test) between the healthy and allergic donors (Fig. 6A). Next, the capacity of rIL-21, at a concentration of 10 ng/ml, to inhibit or to enhance rIL-4-induced IgE synthesis *in vitro*, was expressed as the percentage of variation of response, as compared to that obtained in rIL-4 alone. As shown in Figure 6A, levels of rIL4-induced IgE production *in vitro* did not differ significantly (p=0.56, Mann-Whitney test) between the healthy and allergic donors. Similarly, the rIL-21-induced modulatory effects on rIL-4-induced IgE production did not differ in a statistically significant manner between the two groups (Fig. 6B), indicating that the latter effects are not linked to the presence of atopy. It was also determined whether the observed differential sensitivity to the IL-21 modulating effects on IL-4-induced IgE synthesis was associated with a genetic polymorphism in the *IL-21R* gene. This analysis used PBMC from a total of 7 healthy and 17 atopic donors, genotyped for the presence or absence of the T-83C mutant IL-21R gene,

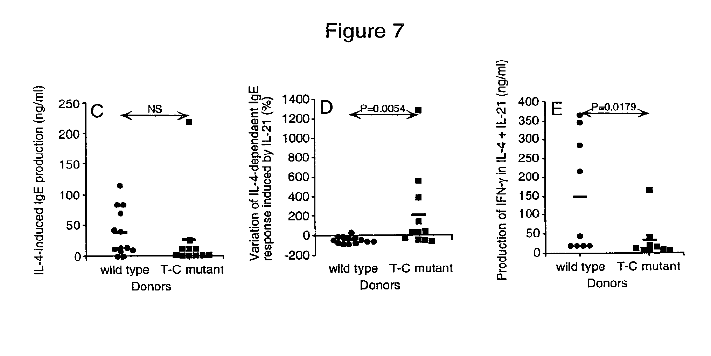

Results from sequencing analysis showed that 13/24 donors were homozygous for the wild-type allele of the *IL-21R* gene, whereas 11/24 donors were heterozygous, expressing one copy of the mutated gene (Table 1). None of the donors, carrying this polymorphism, expressed two copies of the mutant allele. Again, no statistically significant differences could be observed between the two groups of donors with respect to the capacity of IL-4 to promote IgE synthesis *in vitro* (Fig. 7A). However, as shown in Fig. 7B, the variation of rIL-4-induced IgE production, mediated by rIL-21, was significantly different between the donors expressing the wild type alleles and those carrying a mutant copy of the *IL-21R* gene (p=0.0054 Mann-Whitney test). Hence, *in vitro* IgE synthesis by PBMC of 12 out of the 13 donors (92%) homozygous for the wild type allele, was already inhibited by 10 ng/ml rIL-21, whereas this concentration of rIL-21 did not inhibit, but rather enhanced rIL-4-dependent IgE production *in vitro* by 7 out of the 11 individuals (64%) who carried one copy of polymorphic gene. Finally, rIL-21 induced higher levels of IFN-γ production by PBMC from wild type individuals, as compared to those carrying the IL-21R polymorphic variant (Fig. 7C : p=0.018 Mann-Whitney test). Taken together, these results suggest that the presence of at least one copy of the T-83C mutation of the *IL-21R* gene is associated with a decreased capacity of IL-21 to induce the production of IFN-γ, resulting in a reduced sensitivity of the individuals carrying this allele to the inhibitory effect ofIL-21 on the production of IgE.

**Table 1**

| Donors | Age | Sex | Atopy^{b} | L-21R gene^{c} |
|---|---|---|---|---|
| | | | | |

| Healthy | | | | |
|---|---|---|---|---|
| 1 | 51 | M | None | wild type |
| 2 | 40 | M | None | wild type |
| 3 | 27 | M | None | wild type |
| 4 | 60 | M | None | wild type |
| 5 | 50 | M | None | variant |
| 6 | 24 | F | None | wild type |
| 7 | 32 | F | None | wild type |

| Atopic | | | | |
|---|---|---|---|---|
| 1 | 65 | F | Latex, Dp, Gr | wild type |
| 2 | 36 | F | Peni, DHE | wild type |
| 3 | 44 | F | Peni, Dp | wild type |
| 4 | 51 | F | Peni, Dp, latex | wild type |
| 5 | 46 | M | Gr | wild type |
| 6 | 29 | M | Gr, AINS | wild type |
| 7 | 35 | F | Peni, Dp | wild type |
| 8 | 57 | M | Latex, Dp, Gr | variant |
| 9 | 35 | F | Latex, Dp, Gr, Ckrh | variant |
| 10 | 45 | M | Dp | variant |
| 11 | 59 | M | Peni | variant |
| 12 | 44 | F | Peni | variant |
| 13 | 58 | F | Latex, Dp, Gr | variant |
| 14 | 26 | F | Latex, peni, Dp, Gr | variant |
| 15 | 42 | F | Latex, Dp, Ckrh | variant |
| 16 | 28 | M | Gr | variant |
| 17 | 54 | F | Latex, Gr, quinol | variant |

| | | | | |
|---|---|---|---|---|
| ^{a}Genomic DNA was isolated from peripheral blood of 7 healthy non allergic donors and 17 atopic patients and the IL-21R gene was analyzed by sequencing as described above. ^{b} Dp, *Dermalophagoïdes pteronyssinus;* Gr, grass pollen; Ckrh, cockroach; Peni, penicillin; quinol, quinolone. ^{c}wild type: donors carrying both wild-type alleles of the IL-21R gene; variant: donors heterozygous, carrying one copy of the T-83C allele. | | | | |

From the foregoing, it will be appreciated that, although specific embodiments of the invention have been described herein for purposes of illustration, various modifications may be made without deviating from the spirit and scope of the invention. Accordingly, the invention is not limited except as by the appended claims.

### CLAUSES

1. A method of predicting therapeutic response to IL-21 in an individual in need of IL-21 therapy comprising obtaining a biological sample from the patient and detecting the genotype of the individual's *IL-21* receptor gene.
2. The method of clause 1, where the genotype of the individual's *IL-21* receptor gene comprises one allelic variant.
3. The method of clause 1, where the genotype of the individual's *IL-21* receptor gene comprises two allelic variants.
4. The method of clause 1, where the genotype of the individual's *IL-21* receptor gene comprises an IL-21 haplotype.
5. The method of clauses 2 or 3, where the allelic variant is a T-83C variant.
6. A method of selecting a therapeutic regimen for use of IL-21 comprising obtaining a biological sample from an individual; detecting the genotype of the individual's *IL-21* receptor gene; and determining a therapeutically effective dose of an IL-21 composition wherein the IL-21 dose differs from the dose given to individuals that are homozygous for the wildtype *IL-21* receptor gene.
7. The method of clause 6, where the therapeutically effective dose is an optimal immunological dose.
8. The method of clause 6, where the genotype of the individual's *IL-21* receptor gene comprises two allelic variants.
9. The method of clause 6, where the genotype of the individul's *IL-21* receptor gene comprises one allelic variant.
10. The method of clauses 8 or 9, where the allelic variant is a T-83C variant.
11. The method of clause 6, wherein the therapeutically effective dose is administered to the individual.
12. The method of clause 6, where the genotype of the individual's *IL-21* receptor gene comprises an IL-21 haplotype.
13. An *in vitro* method for predicting therapeutic response to IL-21 in an individual in need of IL-21 therapy, comprising, in an isolated biological sample, detecting the genotype of the individual's *IL-21* receptor gene.
14. A method for selecting a therapeutic regimen for use of IL-21, comprising the method according to clause 13, and further comprising determining a therapeutically effective dose of an IL-21 composition wherein the IL-21 dose differs from the dose given to individuals that are homozygous for the *wildtype IL-21* receptor gene.
15. A method according to clause 13 or 14, wherein the genotype of the individual's *IL-21* receptor gene comprises two allelic variants.
16. A method according to clause 13 or 14, wherein the genotype of the individual's *IL-21* receptor gene comprises one allelic variant.
17. A method according to clause 15 or 16, wherein the allelic variant is a T-83C variant.

## Claims

1. An *in vitro* method of predicting a differential therapeutic response to IL-21 in an individual in need of IL-21 therapy, comprising detecting, in a biological sample from the patient, an allelic variant of the individual's IL-21 receptor gene.

2. An *in vitro* method of selecting a differential therapeutic regiment for use of IL-21 in treating an individual in need of IL-21 therapy, comprising:
detecting in a biological sample from an individual, an allelic variant of the individual's IL-21 receptor gene, and
determining a therapeutically effective dose of an IL-21 composition; wherein the IL-32 dose differs from the dose given to individuals that are homozygous for the wild-type IL-21 gene.

3. Th*e in vitro* method of Claim 2 where the therapeutically effective dose is an optimal immunological dose.

4. The *in vitro* method of any of Claims 1-3 wherein the individual has cancer, an infection, an autoimmune disease, or wherein the individual is atopic.

5. Use of a therapeutically effective dose of IL-21 for the manufacture of a medicament for treating an individual in need of IL-21 therapy, wherein the therapeutically effective dose of IL-21 is determined by detecting an allelic variant of the individual's IL-21 receptor gene in a biological sample from an individual, and wherein the therapeutically effective dose of IL-21 differs from the dose given to individuals that are homozygous for the wild-type IL-21 receptor gene.

6. The use of Claim 5 wherein the individual has cancer, an infection, an autoimmune disease, or wherein the individual is atopic.

7. A therapeutically effective dose of IL-21 for use in treating an individual in need of IL-21 therapy, wherein the therapeutically effective dose of IL-21 is determined by detecting an allelic variant of the individual's IL-21 receptor gene in a biological sample from an individual, and wherein the therapeutically effective dose of IL-21 differs from the dose given to individuals that are homozygous for the wild-type IL-21 receptor gene.

8. The therapeutically effective dose of IL-21 for use according to Claim 7, wherein the individual has cancer, an infection, an autoimmune disease, or wherein the individual is atopic.


SEQUENCE LISTING

<110> INSERM

<120> METHODS OF USING IL-21

<130> P29776Ep-D1-PCT-MRM/PJG

<140> EP 06792239.3
<141> 2006-09-22

<150> US 60/719,388
<151> 2005-09-22

<160> 7

<170> FastSEQ for Windows Version 4.0

<210> 1
<211> 17
<212> DNA
<213> Artificial Sequence

<220>
<223> oligonucleotide primer

<400> 1
gacgggccac accatcc                                                17

<210> 2
<211> 19
<212> DNA
<213> Artificial Sequence

<220>
<223> oligonucleotide primer

<400> 2
cggaaggtgg cattggagg                                              19

<210> 3
<211> 25
<212> DNA
<213> Artificial Sequence

<220>
<223> oligonucleotide primer

<400> 3
gctgctgacc gaggcccccc tgaac                                       25

<210> 4
<211> 24
<212> DNA
<213> Artificial Sequence

<220>
<223> oligonucleotide primer

<400> 4
ctccttaatg tcacgcacga tttc                                        24

<210> 5
<211> 19
<212> DNA
<213> Artificial Sequence

<220>
<223> oligonucleotide primer

<400> 5
gcctgctgca tctagtgtc                                                    19

<210> 6
<211> 19
<212> DNA
<213> Artificial Sequence

<220>
<223> oligonucleotide primer

<400> 6
ccgtgcttca tgagaaaga                                                    19

<210> 7
<211> 20
<212> DNA
<213> Artificial Sequence

<220>
<223> oligonucleotide primer

<400> 7
cgtgcttcat gagaaagatg                                                   20